# EUROPEAN PATENT APPLICATION

(11) **EP 4 564 334 A1**
(43) Date of publication of application: **04.06.2025**
(21) Application number: 23845944.0
(22) Date of filing: 08.05.2023
(51) Int. Cl.: G09B 21/00, G01C 21/26, A43B 3/40, A43B 3/44, A43B 3/46, A43B 3/48, G06F 3/01

(54) **FOOT-MOUNTED DEVICE**

(30) Priority: 29.07.2022 JP 2022121751
(71) Applicant: Ashirase, Inc., Minato-ku Tokyo 105-0001 (JP)
(72) Inventor: TOKUDA Ryohei, Nishitokyo-shi, Tokyo 202-0023 (JP); CHINO Wataru, Nishitokyo-shi, Tokyo 202-0023 (JP); TANAKA Yusuke, Nishitokyo-shi, Tokyo 202-0023 (JP)
(74) Representative: Plougmann Vingtoft a/s
(86) International application number: PCT/JP2023/017230
(87) International publication number: WO 2024/024205

(57) **Abstract**

A guidance assistance device S100 comprises a vibration generating device 20 that applies vibration to the foot of a user, a control unit 30 that has a control part 35 connected to the vibration generating device 20, and a wiring 40 for connecting the control part 35 and the vibration generating device 20. The control unit 30 has a housing 310 that accommodates the control part 35, and a clip member 320 that fixes the housing 310 to footwear 50 worn by the user. The wiring 40 extends out from between the housing 310 and the clip member 320 and connects the control part 35 and the vibration generating device 20 while the portion that extends out from between the housing 310 and the clip member 320 forms a loop.

## Description

### TECHNICAL FIELD

The present invention relates to a foot-mounted device, such as a guidance support device, to be worn on a foot of a user.

### BACKGROUND OF THE INVENTION

Conventionally, various navigation systems for assisting a user who moves on foot have been proposed. For example, Patent Document 1 describes a guidance support device that informs a user of a direction that the user should move in by applying vibrations to a foot of the user. In this device, a control unit that controls the device is attached to, for example, a part of footwear.

### PRIOR ART

### PATENT DOCUMENT

Patent Document 1] WO 2021/106770

### SUMMARY OF INVENTION

### PROBLEMS TO BE SOLVED BY THE INVENTION

As noted above, in the technique of Patent Document 1, the control unit is attached to a part of footwear, but the shapes of footwear worn by the user vary. Therefore, conventional configurations have left room for improvement in terms of suitably attaching the control unit to footwear of various shapes. This problem may arise not only in the guidance support device but also, for example, in devices for acquiring data on the user's movement.

The present invention focuses on these points, and its object is to provide a foot-mounted device that can be suitably mounted to footwear of various shapes.

### MEANS FOR SOLVING THE PROBLEM

A foot-mounted device according to a first aspect of the present invention includes: a device that applies vibrations to a foot of a user or acquires data related to motions or biological information of the user; a control unit that has a control part connected to the device; and wiring that connects the control part and the device, wherein the control unit includes: a housing that accommodates the control part; and a fixing implement that secures the housing to footwear worn by the user, wherein the wiring extends through a space between the housing and the fixing implement, and connects the control part and the device in a state where a portion extending from between the housing and the fixing implement forms a loop.

The fixing implement may be detachably attached to the housing.

The foot-mounted device may further include an inner plate inserted into the footwear, wherein the inner plate may include: an inner plate body in which the device is disposed; and an extension part extending in a direction away from the inner plate body and having a distal end portion connected to the housing, and the wiring may be disposed inside the extension part.

The inner plate body may have a shape extending along an outer part of the foot of the user, the extension part may extend upward from the inner plate body, and the housing may be attached to a side of the footwear.

The extension part may include: a first portion extending upward from the inner plate body; a curved portion bent from an upper end of the first portion; and a second portion extending downward from an end of the curved portion, and the housing may be secured to a lower end of the second portion.

The fixing implement may be a clip member that includes: a leaf spring part coupled to the housing; and a plate insertion part that is formed so as to be connected to a part of the leaf spring part on a side opposite to a side connected to the housing, and is inserted between the footwear and the foot of the user.

A first leaf spring part and a second leaf spring part located at positions separated from each other in a direction intersecting an extending direction of the wiring may be provided as the leaf spring part, and the wiring may be disposed between the first leaf spring part and the second leaf spring part.

The housing may include: a coupling recess to which the leaf spring part is coupled; and a guide groove formed to extend toward the coupling recess on a surface of the housing on which the coupling recess is formed, wherein the leaf spring part may be slidably moved along the guide groove so that a distal end portion of the leaf spring part is inserted into and secured to the coupling recess.

In the plate insertion part, a pressing part to be pressed by the user may be formed near a connection portion between the plate insertion part and the leaf spring part, and the fixing implement may be configured so that the leaf spring part is elastically deformed by having the user press the pressing part, whereby a gap between a distal end portion of the plate insertion part and the housing is increased.

The housing may have, on a surface opposite to a side on which the fixing implement is provided, a guide recess that is pressed by the user's other finger while the user presses the pressing part with the finger.

A foot-mounted device according to another aspect of the present invention includes: a device that applies vibrations to a foot of a user or acquirers data related to motions or biological information of the user; a control unit that has a control part connected to the device; and wiring that connects the control part and the device, wherein the control unit includes, a housing that accommodates the control part, and a fixing implement that secures the housing to footwear worn by the user, and the wiring connects the control part and the device in a looped state.

The wiring may be passed between the housing and the fixing implement.

The device may be positioned inside the footwear in a state where the foot-mounted device is in use, and the loop may be formed by a portion of the wiring extending from inside the footwear.

The wiring may further include: a first portion extending in a predetermined direction; a second portion folded back from the first portion and extending so as to face the first portion; and a retainer that defines a distance between the first portion and the second portion.

### EFFECT OF THE INVENTION

According to the present invention, it is possible to provide a foot-mounted device that can be suitably mounted to footwear of various shapes.

### BRIEF DESCRIPTION OF THE DRAWINGS

FIG. 1 shows a state in which a guidance support device is worn on a foot of a user.
FIG. 2 is a perspective view of the guidance support device as seen from the front.
FIG. 3 is a perspective view of the guidance support device as seen from the rear.
FIG. 4 is a view showing an example of a state in which the guidance support device is attached to footwear.
FIG. 5 is a block diagram of the guidance support device.
FIG. 6 is a perspective view of a control unit as seen from the front.
FIG. 7 is a perspective view of the control unit as seen from the back.
FIG. 8 is a perspective view showing a housing and a clip member in a separated state.
FIG. 9 is a view showing movement of the clip member.
FIG. 10 is a schematic view showing a state in which the control unit is attached to the side surface of footwear.
FIG. 11 is a schematic view showing a state in which the control unit is attached to footwear whose height is different from that of the footwear of FIG. 10.
FIG. 12 is a schematic diagram showing another example of arrangement of wiring.
FIG. 13 is a schematic view showing still another example of the arrangement of wiring.
FIG. 14 is a schematic diagram showing an example in which a part of the configuration of FIG. 13 is modified.
FIG. 15 is a schematic view showing an example of a fixing implement.
FIG. 16 is a schematic view showing another example of the fixing implement.
FIG. 17 is a schematic view showing an example of a restrain means.

### DETAILED DESCRIPTION OF THE INVENTION

Hereinafter, embodiments of the present invention will be described with reference to the drawings. A foot-mounted device of the present invention is applicable to various functional devices, provided it includes (i) a predetermined device disposed within footwear and (ii) a control unit connected to the device via wiring. In the following description, a guidance support device for guiding a user will be described as an example of the foot-mounted device.

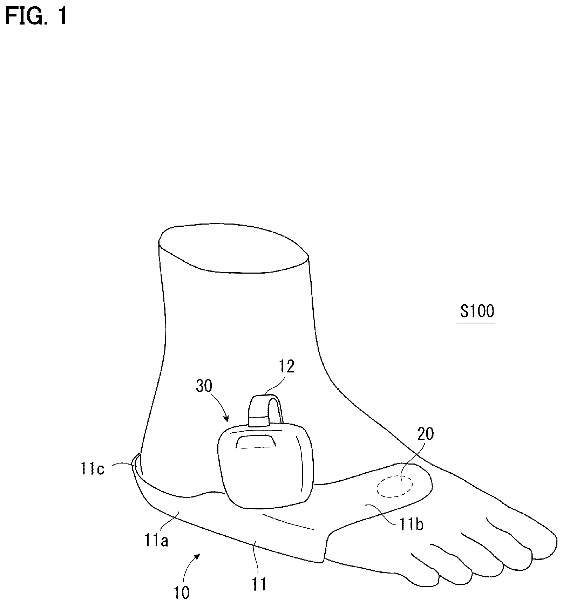

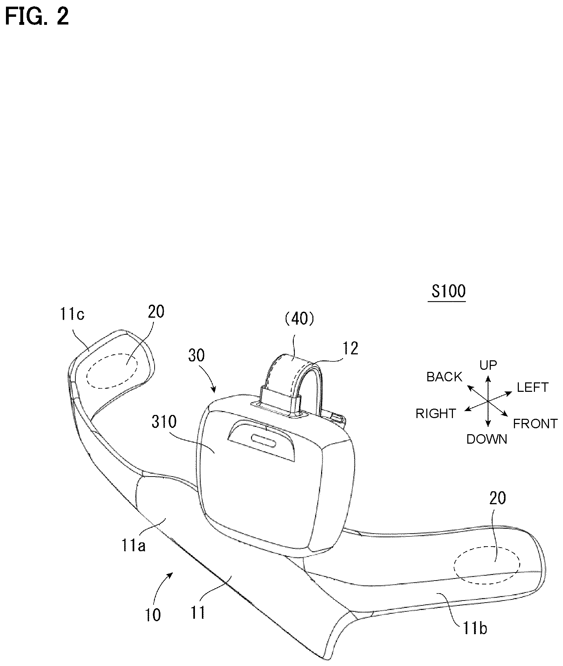

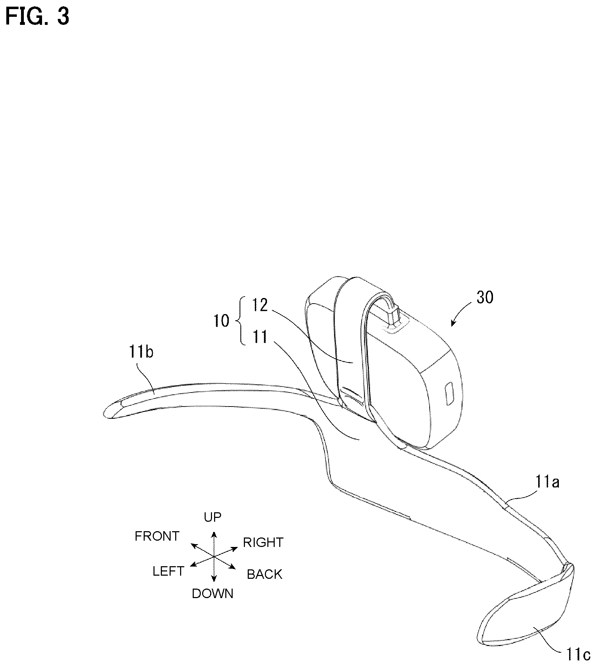

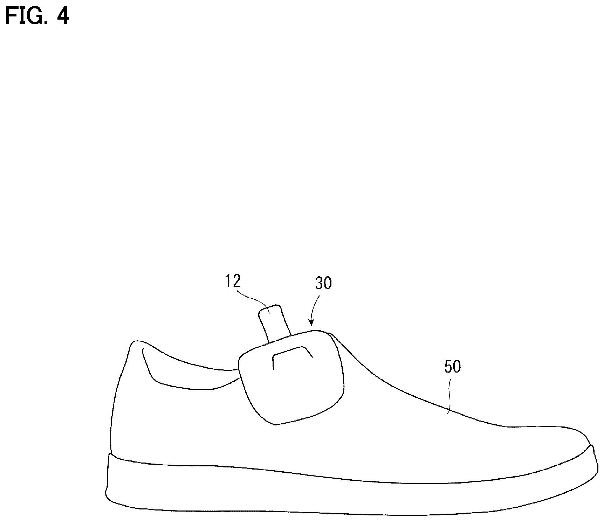

FIG. 1 shows a state in which the guidance support device is worn on a foot of a user. FIG. 2 is a perspective view of the guidance support device as seen from the front. FIG. 3 is a perspective view of the guidance support device as seen from the rear. FIG. 4 shows an example of a state in which the guidance support device is attached to footwear. FIGS. 1 to 4 each show a configuration for the right foot as an example. **In** FIG. 3, a clip member of the control unit is omitted to simplify understanding of the configuration.

**In** the following description, terms indicating directions such as front, back, left, right, up, and down are used, but these terms are not intended to limit the present invention. The "front-back direction" corresponds to the front-back direction of the foot of the user, and the "left-right direction" corresponds to the left-right direction (width direction) of the foot of the user.

### [Outline of the invention]

A guidance support device S100 of the present embodiment is used by being worn on a foot of a user. The guidance support device S100 provides the user with information indicating, for example, a route along which the user should travel by applying vibrations to the foot of the user.

As illustrated in FIG. 2, the guidance support device S100 includes an inner plate 10, a vibration generating device 20, a control unit 30, and an extension part 12 that accommodates wiring 40.

The inner plate 10 is a member that supports the vibration generating device 20, and is attached to the foot of the user who wears footwear 50. The control unit 30 includes a control part that controls the operation of the vibration generating device 20, and the control part and the vibration generating device 20 are electrically connected to each other by the wiring 40. As shown in FIG. 4, the control unit 30 is attached to the side surface of the footwear 50, for example.

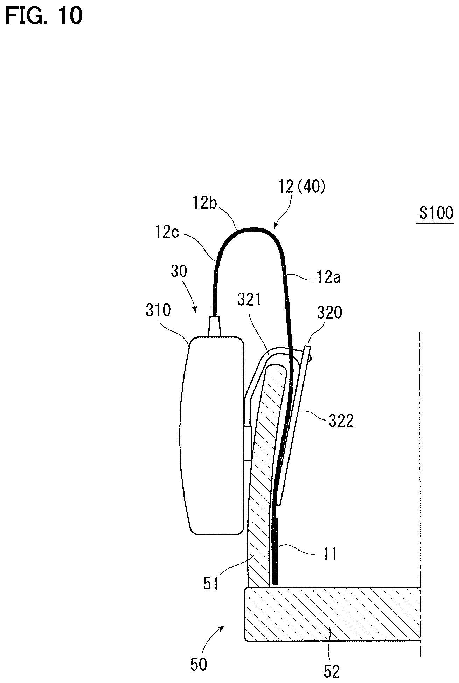

One of the features of the guidance support device S100 of the present embodiment is that the extension part 12, which accommodates the wiring 40 connecting the control part of the control unit 30 and the vibration generating device 20, extends upward from within the footwear 50 and is routed so as to form a loop. Specifically, as an example, the extension part 12 is passed between a housing 310 (FIG. 10, described in detail later) of the control unit 30 and a clip member 320.

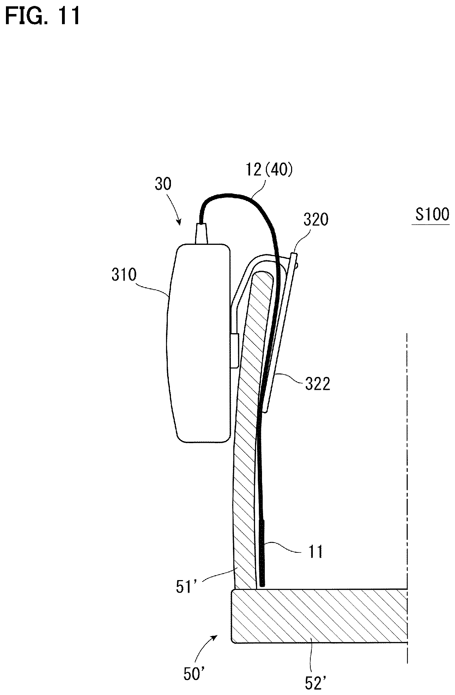

This configuration enables the user to adjust the position of the control unit 30 in the vertical direction. Therefore, in the guidance support device S100, the user can suitably attach the control unit 30 to both a high-top shoe (footwear) 50 (FIG. 11), which is footwear that covers more of the ankle, and a low-top shoe (footwear) 50 (FIG. 10), which is footwear that covers less of the ankle.

### [Configuration of each unit]

### (Electrical configuration of the guidance support device S100)

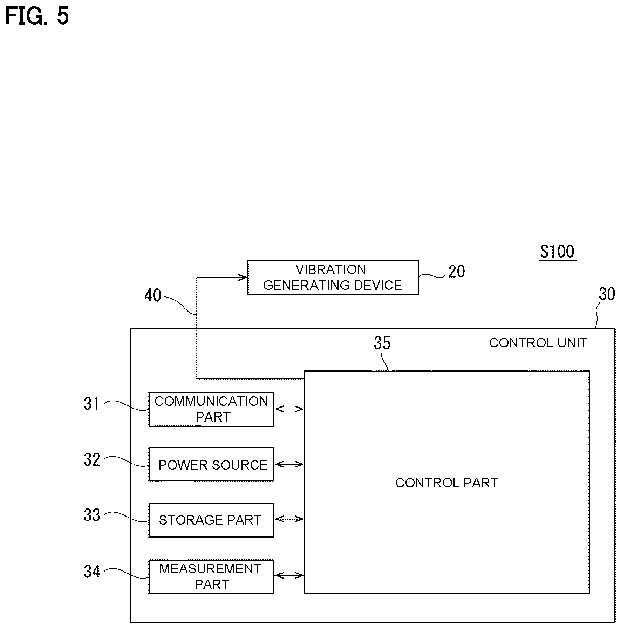

First, an electrical configuration of the guidance support device S100 will be described with reference to FIG. 5. FIG. 5 is a block diagram of the guidance support device S100. FIG. 5 shows the vibration generating device 20, the control unit 30, and the wiring 40. The control unit 30 includes, as electrical components, a communication part 31, a power source 32, a storage part 33, a measurement part 34, and a control part 35.

The communication part 31 is an interface through which the control part 35 exchanges signals with an external device. Specifically, the communication part 31 includes, for example, a wireless communication module and a global navigation satellite system (GNSS) receiving module.

The power source 32 is a battery that supplies power to each unit of the guidance support device S100. The power source 32 is, for example, a secondary battery such as a lithium ion battery. The storage part 33 is a storage device that stores an operating system (OS), application programs, and various types of information referred to when the application programs are executed.

The measurement part 34 includes at least one of an acceleration sensor, a gyro sensor, an azimuth sensor, an atmospheric pressure sensor, or an image sensor. The data related to motions of the user acquired by the measurement part 34 is processed by the control part 35, which is electrically connected to the measurement part 34, for example.

The control part 35 is, for example, a processor such as a central processing unit (CPU), and exhibits various functions by executing programs stored in the storage part 33. The control part 35 communicates with, for example, the user's smartphone via the communication part 31. Specifically, as an example, the control part 35 receives route information transmitted from the smartphone for guiding the user to a predetermined destination, and vibrates one or more vibration generating devices 20 provided in the inner plate 10 (FIG. 2) on the basis of the received route information.

The control part 35 causes the one or more vibration generating devices 20 to operate in a predetermined pattern at a timing when a user who is traveling, for example, needs to make a motion such as turning to the right or going up steps. **In** this way, the user can acquire information indicating a direction that he/she should move in as tactile information.

In the guidance support device S100, by applying vibrations to the foot of the user in coordination with the navigation function of the user's smartphone in this manner, information indicating the direction the user should move in can be provided to the user by information other than visual or auditory information.

The guidance support device S100 may have a function of detecting a gesture of the foot of the user and executing a predetermined command associated with the gesture. Specifically, for example, when the user points the toe of footwear downward and taps the toe against the ground a predetermined number of times, the guidance support device S100 detects these movements. The guidance support device S100 may then execute an operation associated with the gesture pattern (for example, regenerating a route from the position where the user currently is to the destination, and providing the user a notification, by vibrations, indicating in which direction he/she should proceed, in relation to his/her body orientation at that time.)

Not all components shown in FIG. 5 are essential to the present invention, and some of them may be omitted depending on the functionality of the foot-mounted device of the present invention.

### (Structural elements of the guidance support device S100)

As shown in FIGS. 1 and 2, the inner plate 10 is shaped to fit along part of the user's foot, and is inserted into the footwear 50 worn by the user when the guidance support device S100 is in use.

The inner plate 10 is a member made of resin, for example. The inner plate 10 may take any shape as long as it can be inserted between the footwear and the user's foot when the user wears the footwear, and the shape of the inner plate 10 shown in FIGS. 1 to 3 is one example.

Hereinafter, the inner plate 10 will be explained by dividing it into several parts. As shown in FIG. 3, the inner plate 10 includes an inner plate main body 11 and an extension part 12.

The inner plate main body 11 is a portion that is inserted inside the footwear 50. Specifically, the inner plate main body 11 includes an outer part 11a, an instep part 11b, and a heel part 11c.

The outer part 11a extends in the front-back direction along the outer side of the foot of the user (among the sides of the foot of the user, the side opposite to the inner side when the arch side is defined as the inner side) in a state where the inner plate 10 is worn on the foot of the user. The instep part 11b is a portion extending from the front side of the outer part 11a such that it covers the instep of the user's foot. The heel part 11c is a portion curved along the heel of the user's foot from the rear side of the outer part 11a.

As shown in FIG. 2, vibration generating devices 20 are disposed at a plurality of locations in the inner plate 10. Details and placement of the vibration generating devices 20 will be described later.

The extension part 12 is a portion extending in a direction away from the inner plate main body 11 (FIG. 3) such that it protrudes outward from the inside of the footwear 50 in a state where the inner plate 10 is worn on the foot of the user. Specifically, the extension part 12 extends upward from the inner plate main body 11.

The extension part 12 has, as an example, a belt-like shape and is flexible. In the present embodiment, the extension part 12 is a hollow member, and the wiring 40 is passed through the extension part 12. A distal end portion of the extension part 12 is connected to the control unit 30. Specifically, the extension part 12 is connected to a housing 310 of the control unit 30, such that the housing 310 is supported in a suspended manner by the extension part 12.

The vibration generating device 20 is a device having a piezoelectric element or a motor, and generates vibrations in a predetermined pattern in accordance with a control signal from the control part 35. The vibration generating device 20 is positioned inside the footwear in a state where the guidance support device is in use, and operates, for example, with power supplied from the power source 32. The number and placement of the vibration generating devices 20 are arbitrary. In the present embodiment, as shown in FIG. 2, a first vibration generating device 20 is disposed in the instep part 11b, and a second vibration generating device 20 is disposed in the heel part 11c, for example. Although not shown, in addition to the instep part 11b and the heel part 11c, the vibration generating device 20 may also be disposed in the outer part 11a.

Each vibration generating device 20 may be disposed on the inner surface or the outer surface of the member of the inner plate 10, but is disposed inside the member of the inner plate 10 in the present embodiment.

The wiring 40 electrically connects each of the vibration generating devices 20 and the control part 35 of the control unit 30. As an example, one end of the wiring 40 is connected to the vibration generating device 20 inside the member of the inner plate 10. In the present embodiment, the wiring 40 is disposed inside the extension part 12 and is not exposed to outside. The other end of the wiring 40 is connected to the control part 35 in the housing 310.

The wiring 40 and the vibration generating device 20 are not necessarily disposed inside the member of the inner plate 10. However, in the configuration where the wiring 40 and the vibration generating device 20 are disposed inside the inner plate 10 and are not exposed to the outside, as in the present embodiment, the user's comfort of wearing is improved. **In** addition, since the wiring 40 and the vibration generating device 20 do not come into direct contact with the user's foot or footwear, it is less likely that the wiring 40 will become disconnected or the vibration generating device 20 will break. With this configuration, there is also the advantage that, for example, deterioration of the wiring 40 or the vibration generating device 20 due to sweat or humidity inside the footwear, failures caused by short circuits from water exposure, or the like are less likely to occur.

It should be noted that the vibration generating device 20 may be disposed at three or more locations in the inner plate 10. The placement of the vibration generating devices 20 is not limited to the example of FIG. 2, and the vibration generating devices 20 may be disposed at any positions.

### (Detailed configuration of the control unit 30)

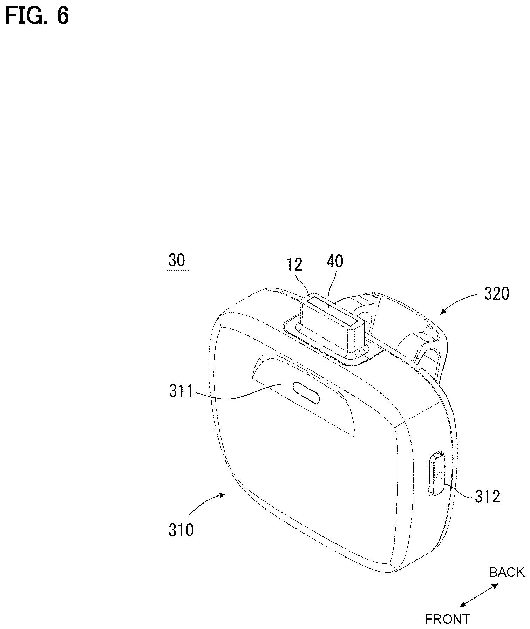

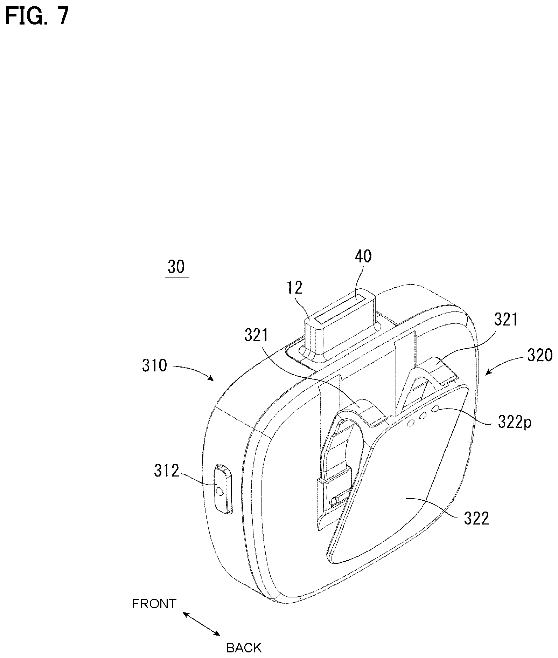

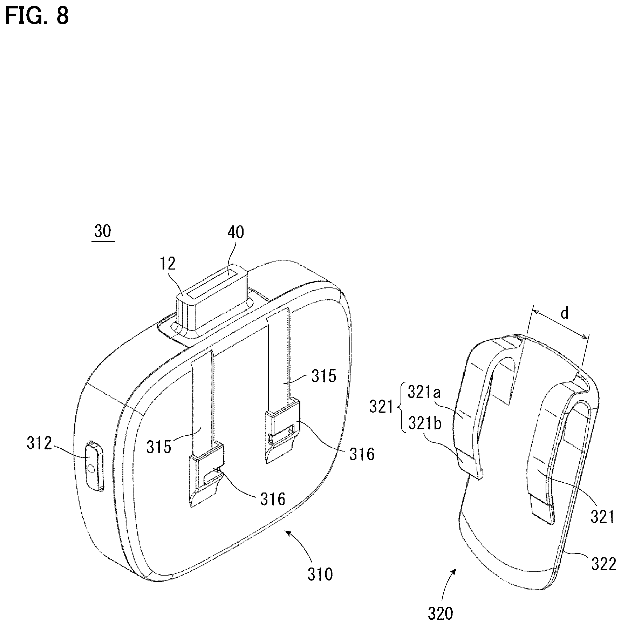

Hereinafter, a detailed configuration of the control unit will be described with reference to FIGS. 6 to 8. FIG. 6 is a perspective view of the control unit as seen from the front. FIG. 7 is a perspective view of the control unit as seen from the back. FIG. 8 is a perspective view showing the housing and the clip member in a separated state. In FIGS. 6 to 8, part of the extension part 12 and the wiring 40 are shown in a cut-off state.

As shown in FIG. 6, the control unit 30 includes the housing 310 and the clip member 320.

The housing 310 is a case that forms an internal space which accommodates the communication part 31, the power source 32, the storage part 33, the measurement part 34, and the control part 35 shown in FIG. 5. The housing 310 is made of resin, for example. The extension part 12 is connected to the upper surface of the housing 310. A recess 311 is formed on the front side of the housing 310. As an example, a switch 312 is provided on the side surface of the housing 310. The switch 312 is, for example, pressed to turn on the power of the guidance support device S100 to put it in an operable state. The function of the recess 311 will be described later together with the description of the clip member 320.

As shown in FIG. 8, a pair of guide grooves 315 and a pair of coupling recesses 316 are formed on the back surface of the housing 310. These structures are used to attach the clip member 320 to the housing 310.

Each guide groove 315 is formed in a straight line. Specifically, the guide groove 315 is formed to extend from the upper surface of the housing 310 toward the corresponding coupling recess 316. One guide groove 315 and the other guide groove 315 are formed parallel to each other. Due to the formation of these guide grooves 315, as described later, the user can slidably move the clip member 320 along the guide grooves 315 and attach the clip member 320 to the housing 310 with a simple operation.

Each coupling recess 316 is located at the end of the corresponding guide groove 315, and is formed in a shape to allow the insertion of a coupling part 321b, described later, at a distal end of a leaf spring part 321 of the clip member 320.

The clip member 320 is a fixing implement for securing the control unit 30 to the footwear 50. The clip member 320 includes the leaf spring part 321 and a plate insertion part 322. The clip member 320 is, for example, a resin molded product in which the leaf spring part 321 and the plate insertion part 322 are integrally formed.

The leaf spring part 321 is a portion that undergoes elastic deformation and functions as a spring. In the present embodiment, a first leaf spring part 321 and a second leaf spring part 321 are provided in the width direction of the plate insertion part 322. The first leaf spring part 321 and the second leaf spring part 321 are located at positions apart from each other in a direction that intersects the extending direction (the vertical direction in the drawing) of the wiring and are spaced by a predetermined distance d (FIG. 8). The extending directions of the first and leaf spring part 321 and the second leaf spring part 321 are parallel to each other, similar to the guide grooves 315.

Specifically, each leaf spring part 321 includes a curved part 321a and the coupling part 321b. The coupling part 321b is a portion at the distal end of the curved part 321a, and is inserted into the corresponding coupling recess 316 of the housing 310.

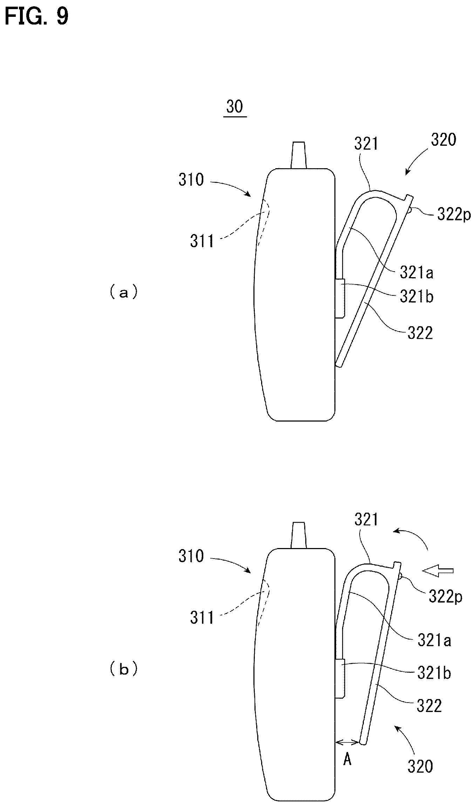

In a state where the clip member 320 is attached to the housing 310 (FIG. 9), the curved part 321a extends in a direction away from the housing 310, with the coupling part 321b as the starting point.

The plate insertion part 322 is a plate-shaped portion designed to be inserted into the footwear 50 of the user. The plate insertion part 322 is formed such that it connects to the end of the leaf spring part 321 that is opposite the coupling part 321b. The plate insertion part 322 of the present embodiment is flat as an example, but a plate insertion part may also be gently curved to follow the contour of the user's foot, for example.

A pressing part 322p, which is pressed by the user, is formed on the surface of the plate insertion part 322 opposite the surface facing the housing 310. The pressing part 322p is, for example, a protrusion. Specifically, as an example, the pressing part 322p is formed in the region of the plate insertion part 322 near a connection portion between the leaf spring part 321 and the plate insertion part 322. As an example, the pressing part 322p is provided at a position that does not exceed the height of the housing 310.

### (Operation of the clip member 320)

FIG. 9 is a view showing movement of the clip member. In the housing 310 and the clip member 320, configured in the manner described above, the user pinches, for example, the recess 311 of the housing 310 and the pressing part 322p of the clip member 320 with his/her fingers while the clip member 320 is in a closed state, as shown in FIG. 9(a).

Then, as indicated by the outlined arrow in FIG. 9(b), the user presses the pressing part 322p toward the housing 310. This causes the curved part 321a of the leaf spring part 321 to elastically deform. Specifically, the leaf spring part 321 deforms such that the curved part 321a moves closer to the housing 310, using the vicinity of a connection portion between the coupling part 321b and the curved part 321a as a fulcrum. As a result, the gap between a distal end portion of the plate insertion part 322 and the housing 310 increases as indicated by a reference numeral A in the drawing. The user can thus attach the control unit 30 to, for example, a part of the footwear 50 with the clip member 320 in an opened state.

According to the configuration of the present embodiment, the leaf spring part 321 of the clip member 320 is deformed such that the vicinity of the connecting portion between the coupling part 321b and the curved part 321a acts as the fulcrum, the pressing part 322p acts as the point of force, and the distal end portion of the plate insertion part 322 acts as the point of action. In the present invention, a leaf spring without the pressing part 322p may be employed. However, according to the configuration in which the clip member 320 is opened by pressing the pressing part 322p, as in the present embodiment, the user does not need to perform an operation of opening the clip member 320 by hooking his/her fingers on the distal end (lower end in the drawing) of the plate insertion part 322 so as to separate the clip member 320 from the housing 310, for example. Therefore, the clip member 320 can be opened with a simple operation.

In addition, in the present embodiment, the pressing part 322p is located near the connection portion between the leaf spring part 321 and the plate insertion part 322, and the pressing part, which acts as the point of force, is not located above the housing 310. Therefore, the clip member 320 can be made compact, contributing to the overall downsizing of the control unit 30. Moreover, in a configuration where the pressing part, acting as the point of force, is located above the housing 310, there is a possibility that the pressing part may contact, for example, the vicinity of the ankle of the user since it is positioned above the housing 310. However, according to the configuration of the present embodiment, such a possibility does not occur, ensuring a comfortable fit.

In addition, in the configuration of the present embodiment, the recess 311 for guiding, which is to be pressed with a finger of the user other than the ones pressing the pressing part 322p, is formed on the surface of the housing 310 opposite to the side on which the clip member 320 is provided. Therefore, when opening the clip member 320, it is easy for the user to intuitively understand that he/she only needs to press the recess 311 and the pressing part 322p of the control unit 30.

### (Replacement and attachment)

The clip member 320 configured as described above is detachable (freely detachable) from the housing 310, and can be replaced with another clip member 320 when the clip member 320 is damaged, for example. When the user replaces the clip member 320, he/she first removes from the housing 310 the clip member 320 that was previously attached to it.

Next, the user attaches a new clip member 320 to the housing 310. Specifically, the user positions the clip member 320 such that the leaf spring part 321 of the clip member 320 is aligned within each guide groove 315 of the housing 310. Then, the user slidably moves the clip member 320 along the extending direction of the guide groove 315 to insert and secure the coupling part 321b into the corresponding coupling recess 316.

As described above, in the configuration of the present embodiment, the clip member 320 can be attached to the housing 310 by positioning the clip member 320 at a predetermined position on the back surface of the housing 310 and then sliding the clip member 320 linearly. Therefore, the clip member 320 can be easily attached.

### (Routing of wiring 40)

Referring to FIG. 10, attachment of the control unit 30 to the footwear 50 will be described. FIG. 10 is a schematic view illustrating a state in which the control unit 30 is attached to the side surface of the footwear 50. The footwear 50 shown in FIG. 10 is, as an example, a low-cut shoe having a low back, and a cross section of a sole part 52 and an upper part 51 is schematically illustrated.

The housing 310 of the control unit 30 is secured to the footwear 50 by the clip member 320 sandwiching a part of the footwear 50, as shown in FIG. 10. **In** the control unit 30 of the present embodiment, in a state where the housing 310 is secured to the footwear 50, the extension part 12, which has the wiring 40 disposed inside, is disposed so as to pass through the space between the housing 310 and the clip member 320 and extend upward (away from the vibration generating device 20). The extension part 12 is disposed so as to pass through the space between a pair of leaf spring parts 321.

A portion of the extension part 12 extending upward from between the housing 310 and the clip member 320 is curved so as to form a loop. The distal end portion of the extension part 12 is connected to the housing 310, and the wiring 40 inside the extension part 12 is connected to the control part inside the housing 310. Specifically, the extension part 12 includes a first portion 12a extending upward from the inner plate main body 11, a curved portion 12b bent from the upper end of the first portion, and a second portion 12c extending downward from the end of the curved portion 12b. The housing 310 is suspended from the lower end of the second portion 12c. As described above, in the present embodiment, since the housing 310 is held by the extension part 12, which is a resin molded product, the housing 310 is stably held as compared with a configuration in which only the wiring 40 is provided.

According to the guidance support device S100 of the present embodiment, the extension part 12 and the wiring 40 are passed through the space between the housing 310 and the clip member 320 in this manner. Therefore, in the area where the clip member 320 is present, the extension part 12 and the wiring 40 do not come into contact with the user's foot inside the footwear 50. Therefore, the user's comfort of wearing is improved, and disconnection of the wiring 40 is less likely to occur. Furthermore, since the extension part 12 and the wiring 40 are disposed between the pair of leaf spring parts 321, the extension part 12 and the wiring 40 are stably supported by the clip member 320.

### (Attachment of the control unit 30 to footwear 50 of varying heights)

Next, the attachment of the control unit 30 to footwear of varying heights will be described with reference to FIG. 11. FIG. 11 is a schematic view showing a state in which the control unit 30 is attached to footwear that has a different height from the footwear 50 shown in FIG. 10. Footwear 50' in FIG. 11 is, for example, a high-top shoe that covers more of the ankle, and a cross section of a sole part 52' and an upper part 51' is schematically illustrated.

As shown in FIG. 11, since the control unit 30 of the present embodiment is disposed so that the extension part 12 and the wiring 40 form a loop, it can be suitably attached to the footwear 50' of varying heights. In FIG. 11, due to change in the attachment position of the control unit 30, the size of the loop formed by the extension part 12 and the wiring 40 is smaller than that in the state of FIG. 10.

Although one embodiment of the present invention has been described above, the foot-mounted device of the present invention is not limited to the specific configuration described above, as long as it includes a device for applying vibrations or acquiring data related to the user's motions or biological information, a control unit having a control part connected to the device, and wiring. The control unit includes a housing and a fixing implement for securing the housing to footwear, and the wiring passes through the space between the housing and the fixing implement, forming a loop that extends from between the housing and the fixing implement to connect the control part and the device. For example, the wiring 40 does not need to be disposed inside the extension part 12 of the inner plate 10. In addition, the wiring 40 may be directly connected to the housing 310 and the control part 35 inside the housing 310, without the extension part 12. Even in such a configuration, the effect of routing the wiring 40 so as to pass through the space between the housing 310 and the clip member 320 to form the loop can be obtained in the same manner as in the embodiment described above.

It is not necessary to provide a plurality of leaf spring parts 321 as illustrated in FIG. 8. For example, the clip member 320 may have a single leaf spring part having a wide width, as if two leaf spring parts 321 were integrated. In this case, the wiring 40 may pass through an opening formed in part of the leaf spring part.

### (Effect of the present embodiment)

As described above, in the guidance support device S100 of the present embodiment, the wiring 40 is disposed between the housing 310 and the clip member 320, and the wiring 40 is routed in such a way that it forms a loop whose size changes depending on the position of the control unit 30 in the footwear 50. Therefore, the control unit 30 can be suitably mounted to the footwear 50 or 50' of various shapes. In addition, since the wiring 40 is disposed between the housing 310 and the clip member 320, the wiring 40 is less likely to be damaged.

In the guidance support device S100, a housing 310 is attached to the side surface of the footwear 50. The housing 310 may be attached, for example, to a portion of footwear that covers the instep. However, when the housing 310 is attached to the side surface of the shoe, as in the present embodiment, the position of the housing 310 is relatively less likely to shift when the user walks. Therefore, for example, when a sensor or similar device for detecting the user's movement is accommodated in the housing 310, this arrangement is preferable because the accuracy of the acquired data is improved.

It is assumed that the user is likely to feel pain in the foot if the clip member 320 is located in the instep portion, where tightness is relatively high among different positions of the footwear. In contrast, when the housing 310 is attached to the side surface of the footwear, as in the present embodiment, comfort of wearing can be improved while reducing pain felt by the user. In addition, for instance, it is assumed that attaching the control unit 30 to the instep portion where shoelaces are present is relatively difficult for a visually impaired person. However, in the case of attaching the housing to the side surface, there is also an advantage of easier attachment.

Further, in the configuration of the present embodiment, the clip member 320 is replaceable with respect to the housing 310. Therefore, for example, when the clip member 320 is broken, the clip member 320 may be replaced with a new clip member instead of replacing the entire device.

However, in the present invention, the clip member 320 does not need to be a separate member from the housing 310, and both members may be integrally provided.

### <Modification Example 1>

In the first embodiment, the guidance support device S100 that has the vibration generating device 20 is exemplified, but the foot-mounted device of the present invention does not need to include the vibration generating device 20, for example. In the foot-mounted device according to one embodiment of the present invention, the device that is attached to the foot of the user and acquires data related to the user's motions or biological information may be connected to the control part 35 (FIG. 5) by the wiring 40. As an example, such a device may include one or more of various sensors such as an acceleration sensor, a gyro sensor, an azimuth sensor, a temperature sensor, and a pressure sensor.

### <Modification Example 2>

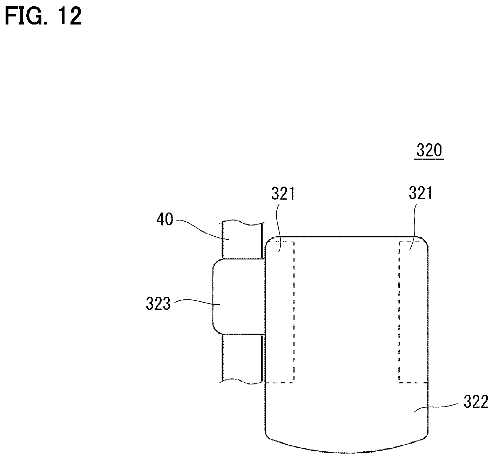

FIG. 12 is a schematic diagram illustrating another example of the arrangement of the wiring 40. In the above embodiment, as an example of the wiring 40 passed through the space between the clip member 320 and the housing 310, a configuration in which the wiring 40 is disposed between the first leaf spring part 321 and the second leaf spring part 321 has been exemplified. However, as shown in FIG. 12, the wiring 40 may be passed through the space between the housing 310 and the fixing implement in a manner of being disposed in a region other than the region between the first leaf spring part 321 and the second leaf spring part 321.

In the example of FIG. 12, a protruding part 323 is formed in the plate insertion part 322 of the clip member 320. The protruding part 323 is, for example, a portion formed so as to protrude outward from the plate insertion part 322. The wiring 40 is passed through the space between the protruding part 323 and the housing (not shown, but located in the direction toward the back of the page in FIG. 12). Even in such a configuration, as shown in FIG. 11, the wiring 40 is passed through the space between the housing 310 and the clip member 320 in a state where the housing 310 and the clip member 320 are viewed from the side. Therefore, the wiring 40 is held in a state of being pressed by the protruding part 323.

The shape of the protruding part 323 itself may be any shape, and the number of the protruding parts 323 provided on the clip member 320 may also be any number. Such a structure that has the function of pressing the wiring 40 may be formed on a member other than the clip member 320.

### <Modification Example 3>

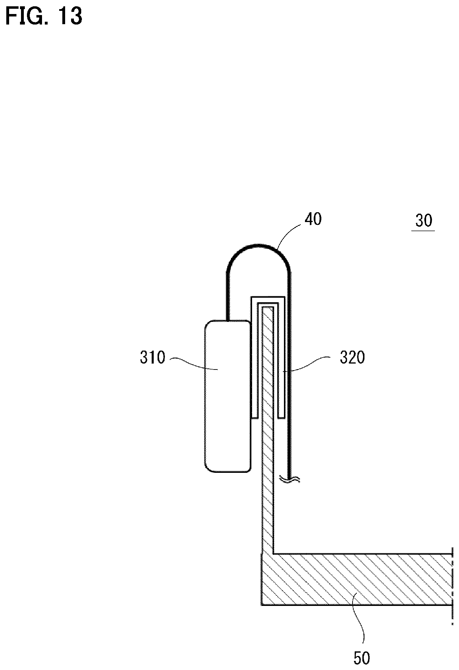

FIG. 13 is a schematic view showing yet another example of the arrangement of the wiring 40. In the above embodiment, the wiring 40 is disposed between the housing 310 and the clip member 320, which secures the housing 310 to the footwear 50. However, as shown in FIG. 13, the wiring 40 may be disposed outside the clip member 320.

In the configuration of FIG. 13, the control unit 30 includes the housing 310 and the clip member 320 which is the fixing implement that secures the housing 310 to the footwear 50. The wiring 40 is disposed in a looped state, but the wiring 40 is disposed at a position that is not between the housing 310 and the clip member 320.

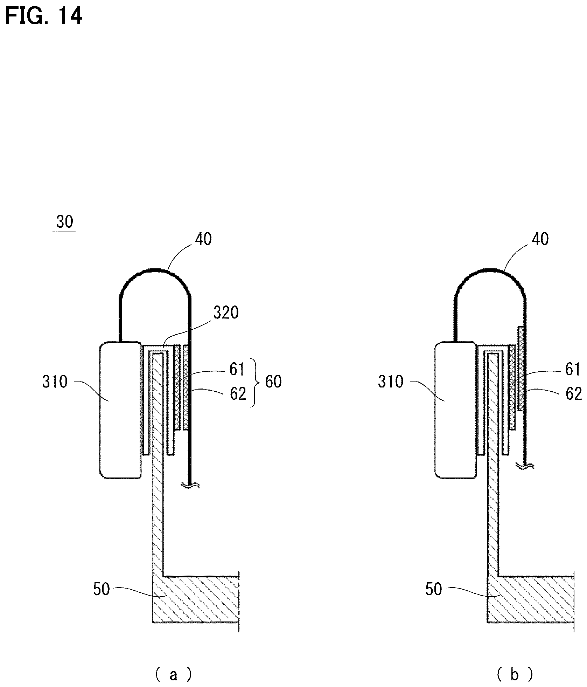

In the case where the wiring 40 is disposed outside the clip member 320 as illustrated in FIG. 13, a configuration illustrated in FIG. 14 may also be employed. FIG. 14 is a schematic diagram showing an example in which a part of the configuration of FIG. 13 is modified. In the configuration of FIG. 14, as an example, a hook-and-loop fastener 60 for securing the wiring 40 to the clip member 320 is provided. In this example, the wiring 40 is attached to the clip member 320, but the wiring 40 may be attached to another fixing implement or another member.

The hook-and-loop fastener 60 includes a first fastener member 61 provided on the clip member 320 and a second fastener member 62 coupled to the first fastener member 61. As an example, the second fastener member 62 is provided on the wiring 40. With this configuration, it is possible to adjust the attachment position of the housing 310 by alternating the relative position of the wiring 40 and the clip member 320 using the hook-and-loop fastener 60. In addition, with the configuration shown in FIG. 14, the wiring 40 is secured to the clip member 320, so that the loop portion of the wiring 40 does not bulge significantly toward the right side of the drawing (the user's foot side). Therefore, the loop portion is prevented from pressing against the foot of the user. In addition, according to such a configuration, there is an advantage in that the user's foot, clothing, or the like is less likely to be caught in the loop portion when the user takes off and puts on the footwear.

### <Modification Example 4>

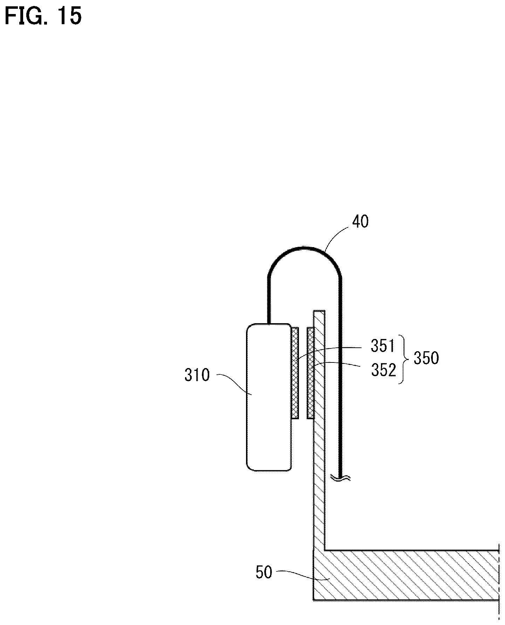

FIG. 15 is a schematic view showing an example of a fixing implement. Although the clip member 320 is illustrated in the above embodiment, the housing 310 may be secured to an object by a hook-and-loop fastener 350. The hook-and-loop fastener 350 may include, for example, a first fastener member 351 provided on the housing 310 and a second fastener member 352 coupled to the first fastener member 351. The second fastener member 352 is attached to the footwear 50, for example. **In** this way, in one aspect of the present invention, the housing 310 may be secured to, for example, the footwear 50 by the hook-and-loop fasteners 350.

### <Modification Example 5>

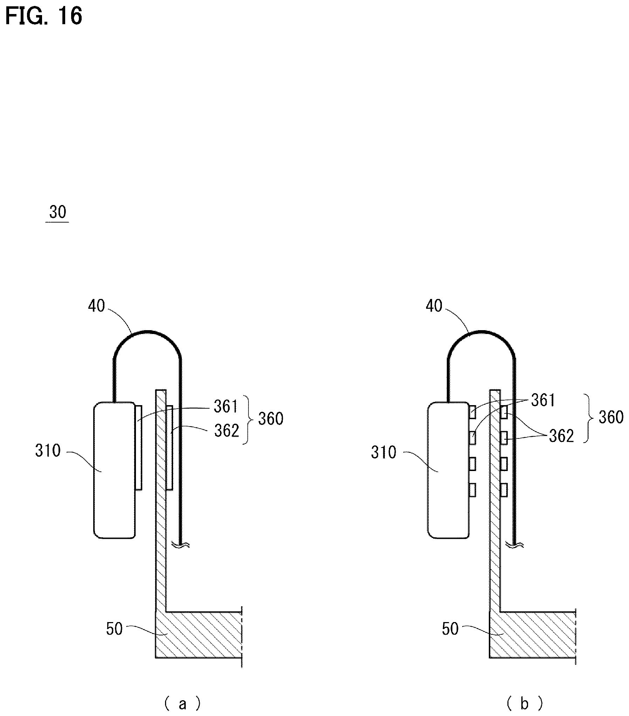

FIG. 16 is a schematic view showing another example of the fixing implement. The housing 310 may be secured to an object by a magnet 360 as shown in FIG. 16(a). The magnet 360 includes, for example, a first magnet body 361 provided on the housing 310 and a second magnet body 362 coupled to the first magnet body 361. The second magnet body 362 may be provided on either an inner surface or an outer surface of the member of the footwear 50, but in this example, it is disposed on the inner surface of the member of the footwear 50. As described above, in one embodiment of the present invention, the housing 310 may be secured to the footwear 50, which is the object, by magnetic force.

Regarding the magnet 360, as illustrated in FIG. 16(b), a plurality of first magnet bodies 361 and a plurality of second magnet bodies 362 may be disposed. Specifically, in this example, the plurality of first magnet bodies 361 are disposed on the housing 310 along the vertical direction at predetermined intervals. Furthermore, the plurality of second magnet bodies 362 are similarly disposed at predetermined intervals. Although second magnet bodies 362 are disposed on the inner surface of the member of the footwear 50 in FIG. 16(b), the second magnet bodies 362 may be disposed on the outer surface of the member of the footwear 50, or may be disposed on another member different from the footwear 50 (e.g., a member such as a clip attached to the footwear 50). According to the configuration as shown in FIG. 16(b), the housing 310 can be attached while shifting the position of the housing 310 in the vertical direction of the drawing.

### <Modification Example 6>

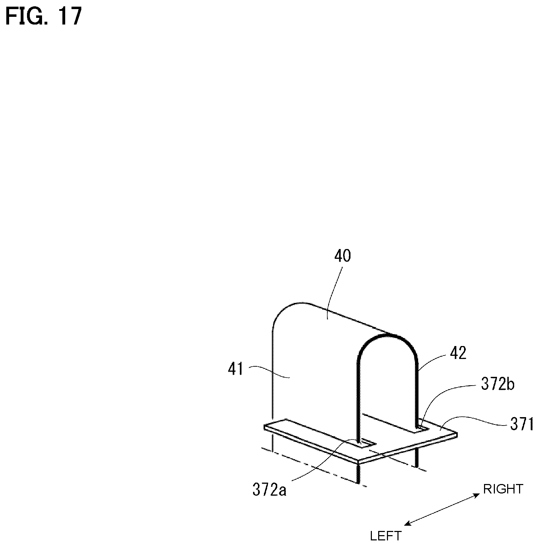

FIG. 17 is a schematic view showing an example of a restrain means. As one example, the left side of the figure corresponds to the outer side of footwear and the right side corresponds to the inner side of footwear. As in the embodiment described above, in the configuration where the wiring 40 is routed from the inner side of the footwear to form, for example, an inverted U-shaped loop, it is assumed that when the loop portion bulges (bulges in the left-right direction in FIG. 17), the wiring 40 may come into contact with the user's foot, reducing the comfort of wearing.

Therefore, a retainer 371 as shown in FIG. 17 may be provided. The retainer 371 is a means to define the shape of the wiring 40. For a more detailed description, it is assumed here that the wiring 40 includes a first portion 41 and a second portion 42, which is folded back from the first portion 41 and extends so as to face the first portion. As an example, the first portion 41 is a portion that extends in the vertical direction in the figure, and the first portion 41 and the second portion 42 are connected by a curved portion bent into an approximately arc shape.

The retainer 371 defines a distance between the first portion 41 and the second portion 42. Specifically, the retainer 371 has a through part 372a for the first portion 41 to pass through and a through part 372b for the second portion 42 to pass through. The distance between the through part 372a and the through part 372b is set to a predetermined length in advance. The retainer 371 may be a metal member or a resin member. Such a retainer 371 may be provided as a part of the clip member 320 or as a part of another fixing implement. In addition, the retainer 371 may be used in combination with the configurations as shown in FIGS. 15 and 16, for example.

Although some examples of the fixing implement have been described above with reference to FIGS. 14 to 16, a fixing implement including a pin and a pin receiver for receiving the pin may be used as the fixing implement. An example of the restrain means is shown with reference to FIG. 17. In consideration of the function of the restrain means for defining the shape of the wiring 40, the hook-and-loop fastener 60 or the like as shown in FIG. 14 has both the function as the fixing implement and the function as the restrain means, for example.

In the above description, the wiring 40 is exemplified as forming the inverted U-shaped loop as an example, but the "loop" may take any shape and is not necessarily limited to the inverted U-shape. The "loop" may be, for example, a form in which the wiring 40 is wound in an annular shape one or more turns, or a form in which the wiring is folded in a zigzag manner. In addition, although the wiring 40 having a flat cross-sectional shape is exemplified above, the cross-sectional shape of the wiring 40 may be any shape and is not limited to a specific shape. A plurality of wirings 40 may be provided. In FIGS. 12 to 17, only the wiring 40 is illustrated, but the wiring 40 may be disposed inside the extension part 12.

The present disclosure is explained based on the exemplary embodiments. The technical scope of the present disclosure is not limited to the scope explained in the above embodiments and it is possible to make various changes and modifications within the scope of the disclosure. For example, all or part of the apparatus can be configured with any unit which is functionally or physically dispersed or integrated. Further, new exemplary embodiments generated by arbitrary combinations of them are included in the exemplary embodiments. Further, effects of the new exemplary embodiments brought by the combinations also have the effects of the original exemplary embodiments. Hereinafter, such variation examples will be described.

### [Description of Symbols]

10 Inner plate
11 Inner plate body
11a Outer part
11b Instep part
11c Heel part
12 Extension part
20 Vibration generating device
30 Control unit
31 Communication part
32 Power source
33 Storage part
34 Measurement part
35 Control part
40 Wire
41 First portion
42 Second portion
50 Footwear
50' Footwear
51 Upper part
51' Upper part
52 Sol part
52' Sol part
60 Hook-and-loop fastener
61 First fastener member
62 Second fastener member
310 Housing
311 Recess
312 Switch
315 Guide groove
316 Coupling recess
320 Clip member
321 Plate spring part
321a Curved part
321b Coupling part
322 Plate insertion part
322p Pressing part
323 Protruding part
350 Hook-and-loop fastener
351 First fastener member
352 Second fastener member
360 Magnet
361 First magnet body
362 Second magnet body
371 Retainer
372a Through part
372b Through part
S100 Guidance support device

## Claims

1. A foot-mounted device comprising:
a device that applies vibrations to a foot of a user or acquires data related to motions or biological information of the user;
a control unit that has a control part connected to the device; and
wiring that connects the control part and the device, wherein
the control unit includes:
a housing that accommodates the control part; and
a fixing implement that secures the housing to footwear worn by the user, wherein
the wiring extends through a space between the housing and the fixing implement, and connects the control part and the device in a state where a portion extending from between the housing and the fixing implement forms a loop.

2. The foot-mounted device according to claim 1, wherein
the fixing implement is detachably attached to the housing.

3. The foot-mounted device according to claim 1 or 2 further comprising:
an inner plate inserted into the footwear,
wherein
the inner plate includes:
an inner plate body in which the device is disposed; and
an extension part extending in a direction away from the inner plate body and having a distal end portion connected to the housing, and
the wiring is disposed inside the extension part.

4. The foot-mounted device according to claim 3, wherein
the inner plate body has a shape extending along an outer part of the foot of the user,
the extension part extends upward from the inner plate body, and
the housing is attached to a side of the footwear.

5. The foot-mounted device according to claim 3, wherein
the extension part includes:
a first portion extending upward from the inner plate body;
a curved portion bent from an upper end of the first portion; and
a second portion extending downward from an end of the curved portion, and the housing is secured to a lower end of the second portion.

6. The foot-mounted device according to claim 1 or 2, wherein
the fixing implement is a clip member that includes:
a leaf spring part coupled to the housing; and
a plate insertion part that is formed so as to be connected to a part of the leaf spring part on a side opposite to a side connected to the housing, and is inserted between the footwear and the foot of the user.

7. The foot-mounted device according to claim 6, wherein
a first leaf spring part and a second leaf spring part located at positions separated from each other in a direction intersecting an extending direction of the wiring are provided as the leaf spring part, and
the wiring is disposed between the first leaf spring part and the second leaf spring part.

8. The foot-mounted device according to claim 6, wherein
the housing includes:
a coupling recess to which the leaf spring part is coupled; and
a guide groove formed to extend toward the coupling recess on a surface of the housing on which the coupling recess is formed, wherein
the leaf spring part is slidably moved along the guide groove so that a distal end portion of the leaf spring part is inserted into and secured to the coupling recess.

9. The foot-mounted device according to claim 6, wherein
in the plate insertion part, a pressing part to be pressed by the user is formed near a connection portion between the plate insertion part and the leaf spring part, and
the fixing implement is configured so that the leaf spring part is elastically deformed by having the user press the pressing part, whereby a gap between a distal end portion of the plate insertion part and the housing is increased.

10. The foot-mounted device according to claim 9, wherein
the housing has, on a surface opposite to a side on which the fixing implement is provided, a guide recess that is pressed by the user's other finger while the user presses the pressing part with the finger.

11. A foot-mounted device comprising:
a device that applies vibrations to a foot of a user or acquirers data related to motions or biological information of the user;
a control unit that has a control part connected to the device; and
wiring that connects the control part and the device, wherein
the control unit includes,
a housing that accommodates the control part, and
a fixing implement that secures the housing to footwear worn by the user, and the wiring connects the control part and the device in a looped state.

12. The foot-mounted device according to claim 11, wherein
the wiring is passed between the housing and the fixing implement.

13. The foot-mounted device according to claim 11 or 12, wherein
the device is positioned inside the footwear in a state where the foot-mounted device is in use, and
the loop is formed by a portion of the wiring extending from inside the footwear.

14. The foot-mounted device according to claim 11 or 12, wherein
the wiring further includes:
a first portion extending in a predetermined direction;
a second portion folded back from the first portion and extending so as to face the first portion; and
a retainer that defines a distance between the first portion and the second portion.
